# FASCICULE DE BREVET EUROPEEN

(11) **EP 2 818 308 B1**
(45) Date de publication et mention de la délivrance du brevet: **17.08.2016**
(21) Numéro de dépôt: 14173386.5
(22) Date de dépôt: 23.06.2014
(51) Int. Cl.: B29D 23/00, A61M 39/10

(54) **Procédé de fabrication d'un raccord**
Verfahren zur Herstellung eines Anschlusses
Method for manufacturing a coupling

(30) Priorité: 25.06.2013 FR 1356082
(43) Date de publication de la demande: 31.12.2014
(73) Titulaire: Technoflex, 64210 Bidart (FR)
(72) Inventeur: Angot, Maxime, 64210 Ahetze (FR); Capitaine, François, 64600 Anglet (FR)
(74) Mandataire: Ipside

(56) Documents cités:
- EP-A2- 0 490 638
- JP-A- S60 107 322
- US-A- 4 687 432

## Description

### ARRIERE-PLAN DE L'INVENTION

### Domaine de l'invention

La présente invention se rapporte aux récipients souples à usage médical et notamment concerne un procédé de fabrication d'un raccord pour de telles poches souples.

Elle vise encore une installation pour la mise en oeuvre de ce procédé de fabrication d'un raccord.

### Arrière-plan technologique

On connaît des éléments d'accès au contenu d'une poche souple destinée à contenir un liquide à usage médical pour assurer le remplissage et la vidange de cette poche.

Un tel élément d'accès prend généralement la forme d'une cheminée, laquelle est formée par l'insertion d'une portion de tube entre les deux parois de matière plastique inerte constitutives de la poche souple et assemblage de l'ensemble ainsi obtenu par soudure au moins des bords périphériques de ces parois.

Une extrémité de cette portion de tube est en communication de fluide avec le volume intérieur de la poche souple tandis que son autre extrémité est placée à l'extérieur de la poche.

Pour établir une communication de liquide entre l'intérieur de la poche souple et l'extérieur, un opérateur n'a alors plus qu'à introduire manuellement l'extrémité d'un connecteur dans cette cheminée.

Pour assurer l'étanchéité de la liaison entre l'extrémité de ce connecteur et la cheminée, il est communément prévu de disposer un jonc sur la paroi intérieure de la cheminée délimitant le canal de passage du liquide (voir par exemple EP0490638).

Ce jonc qui forme alors saillie de la paroi intérieure de la cheminée dans ledit canal, assure un contact linéaire angulaire entre cette paroi et le connecteur.

Outre l'étanchéité de l'assemblage, ce contact limite également les efforts requis au serrage lors de l'assemblage de ces éléments.

La fabrication d'un tel jonc dans le canal intérieur de la cheminée est toutefois rendue difficile par la nature même des matériaux mis en oeuvre dans le domaine médical.

En effet, ces connecteurs sont généralement obtenus par injection d'une matière thermoplastique dans un moule, des broches étant mises en oeuvre pour délimiter le canal intérieur de circulation des liquides dans ces connecteurs et définir le jonc.

Ainsi si le positionnement d'un tel jonc dans le canal d'une cheminée formée à partir d'une matière thermoplastique relativement rigide telle que le polypropylène (PP), est réalisable, il devient laborieux pour des matières thermoplastiques souples et/ou collantes telles que l'éthylène-acétate de vinyle (EVA) ou des dérivés d'EVA en particulier ceux conformes aux prescriptions de la pharmacopée.

On observe, en effet, lors du retrait des broches, un arrachement de matière ou l'apparition de déformations permanentes nécessitant le retrait de la cheminée ainsi formée.

Or, ces matières thermoplastiques telles que l'EVA tendent à s'imposer vis-à-vis des autres matières dans la fabrication des éléments pour le stockage et le transfert de liquide à usage médical en raison de leur inertie vis-à-vis des solutions médicales, de leur résistance à la stérilisation par rayonnement et de leur tenue à basse température.

De plus, la réalisation de tel raccord en EVA serait particulièrement intéressante car leur disponibilité autoriserait la fabrication de poche souple à usage médical réalisées intégralement dans la même matière thermoplastique, et par conséquent, rendrait leur assemblage plus aisé.

La présente invention vise à pallier ces divers inconvénients en proposant un procédé de fabrication d'un raccord pour poche souple à usage médical particulièrement simple dans sa conception et dans son mode opératoire, originale dans son approche et économique, permettant la réalisation d'un jonc sur la paroi intérieure du raccord sans aucune dégradation de celui-ci.

Un autre objet de la présente invention est une installation originale dans son architecture permettant de réaliser industriellement ces raccords avec des temps de production limités.

### BREVE DESCRIPTION DE L'INVENTION

A cet effet, l'invention concerne un procédé de fabrication d'un raccord pour un récipient à usage médical destiné à l'administration de fluide, ledit raccord comprenant un corps principal définissant un canal intérieur pour le passage d'un fluide et au moins un bourrelet continu ou discontinu, formant saillie à l'intérieur dudit canal.

Selon l'invention, on réalise au moins les étapes suivantes:
a) on prend un conduit tubulaire dépourvu de bourrelet formant saillie à l'intérieur dudit canal,
b) on introduit au moins partiellement dans ledit conduit tubulaire ainsi formé un mandrin présentant sur sa surface externe au moins une gorge annulaire continue ou discontinue et dont les dimensions et la forme correspondent à celles d'un bourrelet continu ou discontinu à former dans le canal intérieur dudit conduit,
c) on chauffe en partie seulement ledit conduit tubulaire, ladite partie comprenant au moins la portion dudit conduit placée au niveau de ladite au moins une gorge lorsque ledit mandrin a été positionné dans ledit conduit tubulaire, de sorte que la matière dudit conduit tubulaire remplisse ladite au moins une gorge.

Bien entendu, la section transversale droite de cette gorge peut ne pas être de forme ronde ou sensiblement ronde mais peut au contraire présenter une ou plusieurs parois planes, arrondies, bombées, elliptiques, ou autre, et des combinaisons de ces éléments.

De manière plus générale, le mandrin comporte sur sa surface externe une gorge continue ou discontinue qui est l'empreinte, ou le négatif, de l'objet à former en saillie sur la paroi intérieure du raccord délimitant le canal de passage de fluide. Ainsi, pour définir un bourrelet, ou jonc, sur cette paroi, la surface externe du mandrin comporte une gorge entourant complètement ou encore décrivant la circonférence de ce mandrin.

Le raccord obtenu selon le procédé de l'invention est particulièrement adapté pour établir une communication de fluide, du type liquide ou mélange liquide/poudre, dans un circuit de transfert de fluide à usage médical.

Par ailleurs, le ou chaque jonc étant obtenu par la déformation plastique de la matière en fusion du conduit tubulaire chauffé localement, le raccord final est d'une seule pièce.

On entend par le "raccord étant d'une seule pièce" que ce raccord est d'un seul tenant et ne résulte donc pas de l'assemblage de pièces initialement distinctes.

A titre purement illustratif, ce raccord peut être réalisé dans une matière thermoplastique médicalement inerte telle que l'EVA, des dérivés de l'EVA, en polyéthylène, des dérivés d'acrylate comme l'éthylène-acrylate de méthyle (EMA), en polyoléfine comprenant un thermoplastique élastomère (TPE) tel que le SEB (copolymère bloc Styrène, Ethylène, Butylène), le SEBS (copolymère bloc Styrène, Ethylène, Butylène, Styrène)...

En outre, le procédé de l'invention est particulièrement rapide à mettre en oeuvre et autorise en conséquence une exploitation industrielle de ces raccords avec des coûts de production faibles.

Dans différents modes de réalisation particuliers de ce procédé, chacun ayant ses avantages particuliers et susceptibles de nombreuses combinaisons techniques possibles:
- on refroidit ledit mandrin avant retrait de ce dernier du canal intérieur dudit conduit tubulaire,
- à l'étape c), on chauffe par une méthode de chauffage choisie dans le groupe comprenant :
- chauffage diélectrique, tel que par chauffage à haute fréquence,
- par rayonnement tel qu'avec des rayons lasers, ou encore
- par contact direct avec des éléments chauffés.

Avantageusement, mettant en oeuvre une méthode de chauffage diélectrique, on utilise ledit mandrin comme une desdites électrodes.
- à l'étape a), on forme un conduit tubulaire comprenant au moins un élément d'obstruction dudit canal, ledit au moins un élément d'obstruction étant sécable pour libérer le passage.

De manière avantageuse, on forme une membrane d'étanchéité à l'intérieur dudit canal permettant de garantir l'asepsie du contenu du récipient auquel le raccord sera assemblé.

De préférence, cette membrane d'étanchéité sera positionnée dans le canal intérieur pour former une butée permettant de stopper le déplacement du mandrin lors de son introduction dans ce canal en vue de positionner ledit ou chaque bourrelet en un ou des points précis de ce canal.

Cette membrane d'étanchéité est destinée à être séparé ultérieurement au moyen d'un trocart pour libérer l'accès au canal intérieur. Cette membrane d'étanchéité est, de préférence, rendue sécable par une ligne de moindre résistance. Elle peut, en outre, comporter une zone de renfort sur son pourtour afin d'empêcher une éventuelle introduction dans le circuit de liquide auquel serait connecté le raccord de l'invention d'un morceau de celle-ci après déchirure.

On s'assure aussi grâce à cette membrane d'étanchéité d'un positionnement idéal de la gorge annulaire par rapport à la deuxième électrode, et par conséquent du positionnement précis de bourrelet à l'intérieur du canal intérieur. Un tel positionnement précis du bourrelet est important pour garantir l'étanchéité de l'assemblage trocart/raccord.

De manière plus générale, on forme un élément d'obstruction à l'extrémité dudit conduit tubulaire et/ou à l'intérieur dudit canal, ce dernier étant alors une membrane d'étanchéité.

L'élément d'obstruction placé à l'extrémité dudit conduit peut être un bouchon, un bouchon quart de tour, un opercule ou encore une combinaison de ces éléments.
- à l'étape a), on réalise un chanfrein à au moins une des extrémités dudit conduit corporel de sorte qu'au moins une desdites extrémités est biseautée et/ou on forme sur la surface externe dudit conduit corporel, un ou plusieurs éléments choisi dans le groupe comprenant un jonc, un filetage, une rainure hélicoïdale, une collerette et un élément de jonction qui peut être intégré dans le récipient souple, cet élément de jonction comportant à chacun de ses bords latéraux une ailette, ces ailettes formant chacune un prolongement de la partie centrale de cet élément de jonction, ...

L'invention vise aussi une installation pour la mise en oeuvre du procédé de fabrication d'un tel que décrit précédemment.

Selon l'invention, cette installation comprend :
- un générateur électrique haute fréquence apte à délivrer une tension à haute fréquence,
- une première électrode tubulaire de diamètre égal ou sensiblement égal au diamètre du canal intérieur dudit conduit tubulaire de manière à pouvoir être introduite dans ledit canal intérieur, ladite première électrode comportant sur sa surface externe au moins une gorge annulaire continue ou discontinue et dont les dimensions et la forme correspondent à celles d'un bourrelet continu ou discontinu à former dans le canal intérieur dudit conduit,
- une deuxième électrode comportant un trou de diamètre inférieur ou égal au diamètre extérieur dudit conduit tubulaire de manière à définir un logement pour recevoir et entourer en partie ledit conduit.

L'installation peut encore comprendre un moyen pour refroidir ladite première électrode tel qu'un système de refroidissement à eau.

De manière avantageuse, le canal intérieur dudit conduit comportant une membrane d'étanchéité sécable, l'extrémité libre de ladite première électrode tubulaire présente une surface de contact non pointue permettant de plaquer celle-ci contre ladite membrane d'étanchéité sans la déchirer.

Cette membrane d'étanchéité joue alors le rôle d'un marqueur ou repère pour placer précisément le bourrelet dans le canal intérieur de sorte à s'assurer de l'étanchéité de l'assemblage trocart/conduit tubulaire.

Le canal intérieur dudit conduit comportant une membrane d'étanchéité sécable, la dimension longitudinale séparant la ou chaque gorge de ladite première électrode de son extrémité libre est au moins égale à la distance devant séparer ladite membrane d'étanchéité du ou de chaque bourrelet pour s'assurer de l'étanchéité de l'assemblage dudit conduit et du trocart correspondant.

### BREVE DESCRIPTION DES DESSINS

D'autres avantages, buts et caractéristiques particulières de la présente invention ressortiront de la description qui va suivre faite, dans un but explicatif et nullement limitatif, en regard des dessins annexés, dans lesquels :
- la figure 1 est une vue partielle et en perspective d'une installation pour la fabrication d'un raccord selon un mode de réalisation particulier de la présente invention, un raccord étant positionné dans cette installation en vue de la formation d'un jonc dans son canal intérieur;
- la figure 2 est une vue partielle et en perspective de l'installation de la Fig. 1 ; la demie-électrode supérieure ayant été omise par souci de clarté ;
- la figure 3 est une vue élargie et en coupe de l'installation de la Fig. 1 ;
- la figure 4 est une vue partielle et en perspective de l'installation de la Fig. 1 ; la demie-électrode supérieure ayant été omise par souci de clarté et le mandrin ayant été retiré du corps du raccord après formation du jonc ;
- la figure 5 est une vue en perspective d'un conduit tubulaire dans lequel un jonc doit être formé selon un mode de réalisation de la présente invention;
- la figure 6 est une vue en coupe longitudinale du conduit tubulaire de la Fig. 5 montrant le canal intérieur de passage de fluide dépourvu de jonc mais obturé par une membrane;

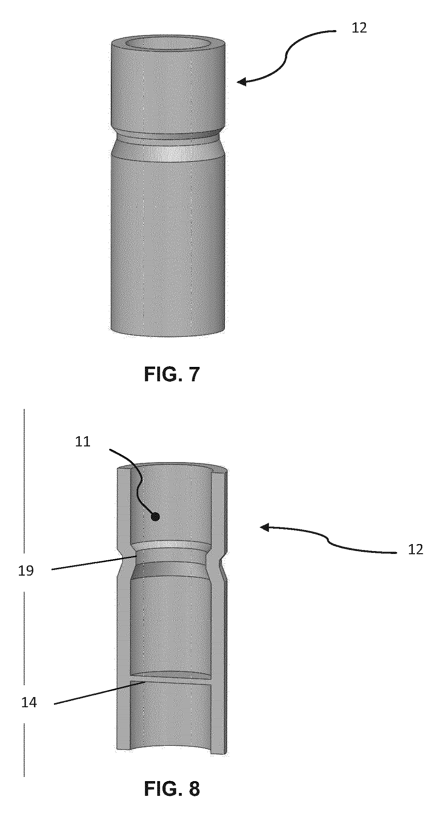

- la figure 7 est une vue en perspective d'un raccord selon un mode de réalisation particulier de la présente invention;
- la figure 8 est une vue en coupe longitudinale du raccord de la Fig. 7 montrant le canal intérieur de passage de fluide obturé par une membrane et comportant un jonc;

### DESCRIPTION DETAILLEE DE MODES DE REALISATION DE L'INVENTION

On notera en premier lieu que les figures ne sont pas à l'échelle.

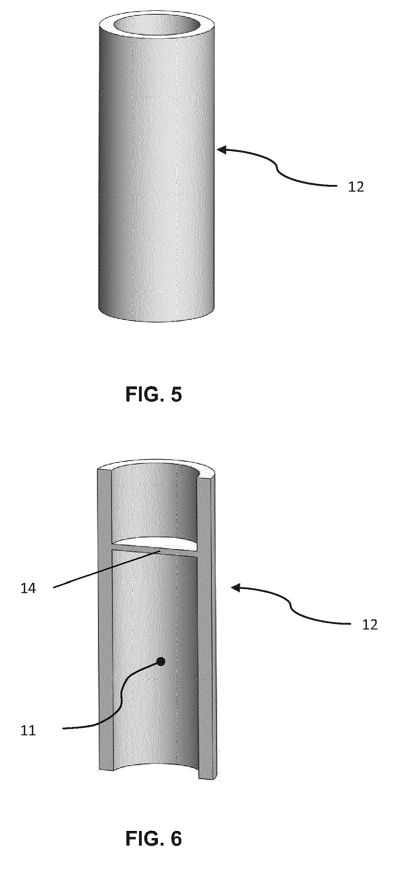

En vue de réaliser un raccord comportant un jonc placé sur sa paroi intérieure délimitant un canal pour le passage d'un fluide, l'opérateur se fournit avec ou fabrique, tout d'abord, un conduit tubulaire 12 dépourvu de jonc faisant saillie dans son canal intérieur 11 (Figures 5 et 6). Ce conduit tubulaire 12 comporte avantageusement une membrane d'étanchéité 14, dont le rôle sera expliqué ci-dessous.

La réalisation de ce conduit tubulaire 12 peut, par exemple, être obtenue par un procédé connu d'injection d'une matière plastique telle que de l'EVA dans un moule.

Une fois ce conduit tubulaire 12 sélectionné ou fabriqué, l'opérateur peut dés lors réaliser un ou plusieurs joncs à l'intérieur de son canal.

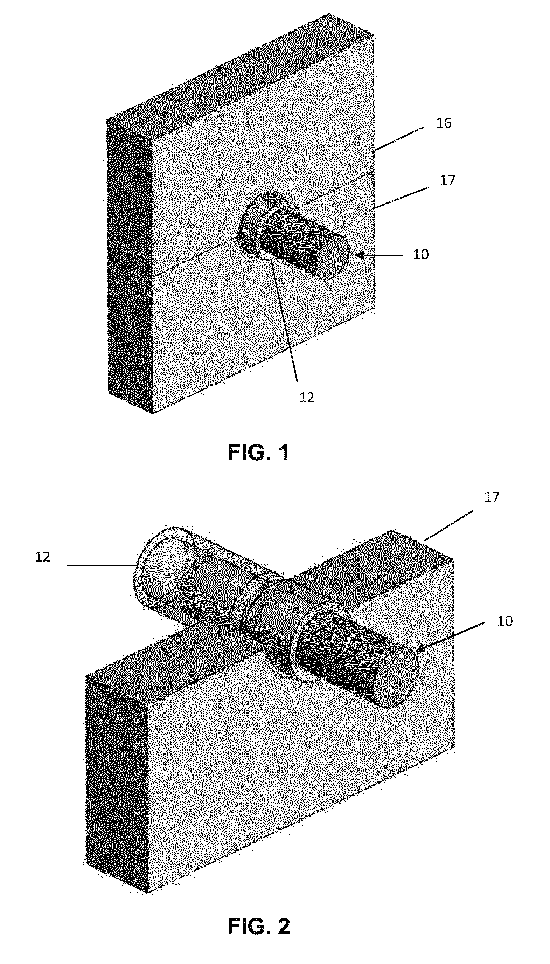

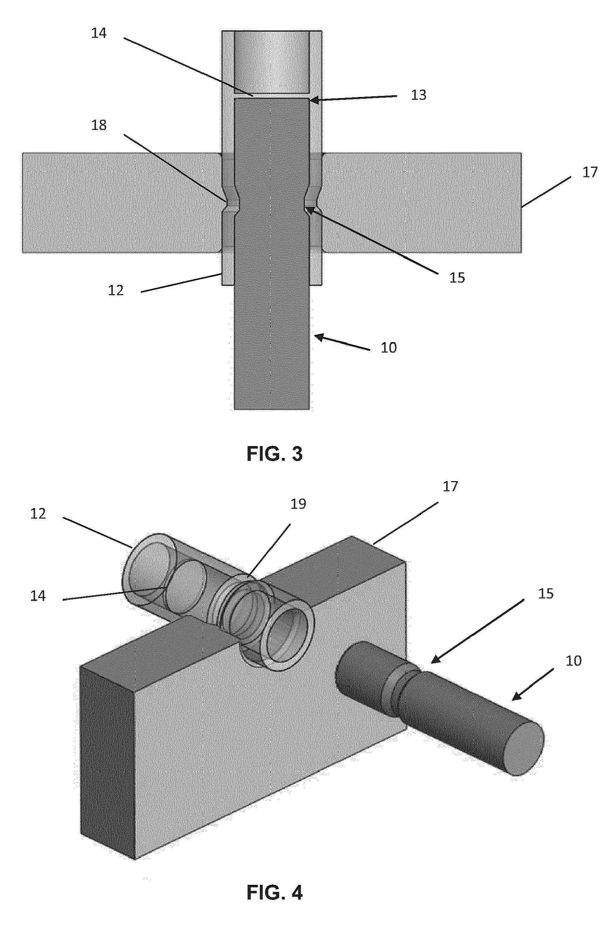

Les figures 1 à 4 montrent une installation pour la fabrication d'un raccord selon un mode de réalisation préféré de l'invention.

Cette installation comprend un générateur électrique haute fréquence (non représenté) capable de délivrer une tension à haute fréquence. Ce générateur est ici capable de fournir des signaux à des fréquences supérieures à quelques dizaines de MHz.

Ce générateur électrique est relié par une ligne de transmission d'énergie (non représentée) à une première électrode tubulaire 10 dont le diamètre est sensiblement égal au diamètre du canal intérieur 11 du conduit tubulaire 12 de manière à être introduite dans ce canal. Cette ligne de transmission d'énergie est ici un câble coaxial.

L'extrémité libre 13 de cette première électrode 10 destinée à être introduite dans le canal intérieur 11 du conduit tubulaire 12, présente une face plane ou sensiblement plane permettant à l'opérateur de ressentir le contact de cette extrémité libre 13 avec la membrane d'étanchéité 14 obturant le canal intérieur 11 du conduit tubulaire 12 sans risque d'endommager celle-ci.

Cette membrane d'étanchéité 14 joue ici le rôle d'un marqueur ou repère, pour positionner précisément la première électrode 10 à l'intérieur du canal intérieur 11.

Ainsi, on s'assure que la gorge annulaire 15 placée sur la surface extérieure de la première électrode 10 est positionnée précisément à une distance d de cette membrane d'étanchéité 14. Le respect de cette distance d est, en effet, nécessaire pour garantir l'étanchéité de la liaison trocart/raccord.

De manière avantageuse, cette gorge annulaire 15 présente une forme et des dimensions qui en font l'empreinte, ou le négatif, du jonc à réaliser sur la paroi intérieure du conduit tubulaire 12, ce jonc étant placé en saillie dans le canal intérieur du raccord. Cette gorge 15 qui décrit le pourtour de la surface extérieure du mandrin 10, présente une section transversale droite comprenant une combinaison d'une paroi centrale plane et parallèle ou sensiblement parallèle à l'axe longitudinal du mandrin 10 et de deux parois d'extrémité planes inclinées placées aux extrémités de la paroi centrale. La paroi plane inclinée qui est la plus proche de la membrane d'étanchéité 14, présente une pente favorisant le retrait du mandrin 10 du conduit tubulaire 12 après formation du jonc.

L'ensemble ainsi assemblé est placé dans une deuxième électrode comportant un trou dans sa partie centrale de diamètre inférieur au diamètre extérieur du conduit tubulaire 12 de manière à recevoir et pincer ce conduit tubulaire 12 en vue de la formation du jonc.

Cette deuxième électrode comprend deux demi-électrodes 16, 17, une 16 de ces demi-électrodes étant mobile par rapport à l'autre demi-électrode 17 de manière à pouvoir être écartée et rapprochée de cette dernière pour assurer le positionnement optimal de l'ensemble conduit tubulaire 12/première électrode 10.

On cherchera, en effet, à ne chauffer que localement le conduit tubulaire 12 pour former le jonc sur la paroi intérieur du conduit tubulaire 12 par transfert de matière en fusion d'une portion uniquement de ce conduit tubulaire 12 dans la gorge annulaire 15.

Ce transfert de matière créé une déformation locale de la surface externe du conduit tubulaire 12 qui n'est en rien gênante pour le bon fonctionnement de ce dernier.

De manière à faciliter la chauffe et le transfert de matière en fusion vers la gorge annulaire 15 du mandrin 10, chaque demi-électrode 16, 17 présente sur la surface délimitant une partie du contour du trou pour recevoir l'ensemble conduit tubulaire 12/première électrode 10, une surface bombée formant saillie pour venir presser cet ensemble au niveau de la gorge 15 du mandrin 10 lorsque ce dernier est en position dans la deuxième électrode. Cette surface bombée qui suit le contour du trou, forme ici une dent 18pour chaque demi-électrode 16, 17. Avantageusement, cette surface bombée a une forme complémentaire de celle de la gorge 15 du mandrin 10.

L'installation comporte encore un moyen pour refroidir (non représenté) la première électrode 10 tel qu'un dispositif de refroidissement à eau refroidie.

Dans un mode de mise en oeuvre de la présente invention, et pour un conduit tubulaire réalisé en EVA, l'apport de chaleur par les deux demi-électrodes 16, 17, 80°C environs pendant trois (3) secondes environs, permet la génération du jonc 19 par la déformation plastique de la matière en fusion de la portion du conduit tubulaire 12 ainsi chauffée.

Si la première électrode 10, par exemple en laiton, n'est pas régulée en température, il faut attendre que le raccord ait refroidi sensiblement avant retrait. Le jonc 19 ainsi réalisé ne risque alors plus d'être détérioré par le frottement de la gorge 15 de la première électrode 10.

## Revendications

1. Procédé de fabrication d'un raccord pour un récipient à usage médical destiné à l'administration de fluide, ledit raccord comprenant un corps principal définissant un canal intérieur (11) pour le passage d'un fluide et au moins un bourrelet (19) continu ou discontinu, formant saillie à l'intérieur dudit canal, comprenant au moins les étapes suivantes:
a) on prend un conduit tubulaire (12) dépourvu de bourrelet (19) formant saillie à l'intérieur dudit canal, ledit canal intérieur dans lequel on forme pour l'obturer, ou étant obturé par, une membrane d'étanchéité sécable,
b) on introduit au moins partiellement dans ledit conduit tubulaire (12) ainsi formé un mandrin présentant sur sa surface externe au moins une gorge (15) annulaire continue ou discontinue et dont les dimensions et la forme correspondent à celles d'un bourrelet (19) continu ou discontinu à former dans le canal intérieur (11) dudit conduit, ladite membrane d'étanchéité étant positionnée dans ledit canal intérieur (11) pour former une butée permettant de stopper le déplacement du mandrin lors de son introduction dans ledit canal en vue de positionner ledit ou chaque bourrelet (19) en un ou des points précis dudit canal,
c) on chauffe en partie seulement ledit conduit tubulaire (12), ladite partie comprenant au moins la portion dudit conduit placée au niveau de ladite au moins une gorge (15) lorsque ledit mandrin a été positionné dans ledit conduit tubulaire (12), de sorte que la matière dudit conduit tubulaire (12) remplisse ladite au moins une gorge (15).

2. Procédé selon la revendication 1, **caractérisé en ce qu'**on refroidit ledit mandrin avant retrait de ce dernier du canal intérieur (11) dudit conduit tubulaire (12).

3. Procédé selon la revendication 1 ou 2, **caractérisé en ce qu'**à l'étape c), on chauffe par une méthode de chauffage choisie dans le groupe comprenant par chauffage diélectrique, par rayonnement et par contact direct avec des éléments chauffés.

4. Procédé selon la revendication 3, **caractérisé en ce que** mettant en oeuvre une méthode de chauffage diélectrique, on utilise ledit mandrin comme une desdites électrodes.

5. Procédé selon l'une quelconque des revendications 1 à 4, **caractérisé en ce qu'**à l'étape a), on réalise un chanfrein à au moins une des extrémités dudit conduit corporel de sorte qu'au moins une desdites extrémités est biseautée et/ou on forme sur la surface externe dudit conduit corporel au moins un des éléments choisi dans le groupe comprenant un ou plusieurs joncs placés en saillie, un élément de jonction qui peut être intégré dans le récipient souple, cet élément de jonction comportant à chacun de ses bords latéraux une ailette, ces ailettes formant chacune un prolongement de la partie centrale de cet élément de jonction, ...

6. Installation pour la mise en oeuvre du procédé de fabrication d'un raccord selon l'une quelconque des revendications 1 à 5, l'installation comprenant:
- un générateur électrique haute fréquence apte à délivrer une tension à haute fréquence,
- une première électrode tubulaire (10) de diamètre égal ou sensiblement égal au diamètre du canal intérieur (11) dudit conduit tubulaire (12) de manière à pouvoir être introduite dans ledit canal intérieur (11), ladite première électrode comportant sur sa surface externe au moins une gorge (15) annulaire continue ou discontinue et dont les dimensions et la forme correspondent à celles d'un bourrelet (19) continu ou discontinu à former dans le canal intérieur (11) dudit conduit,
- une deuxième électrode comportant un trou de diamètre inférieur ou égal au diamètre extérieur dudit conduit tubulaire (12) de manière à définir un logement pour recevoir et entourer en partie ledit conduit, et en ce que
- le canal intérieur (11) dudit conduit comportant une membrane (14) d'étanchéité sécable, l'extrémité libre (13) de ladite première électrode tubulaire (10) présente une surface de contact non pointue permettant de plaquer celle-ci contre ladite membrane d'étanchéité (14) sans la déchirer.

7. Installation selon la revendication 6, **caractérisée en ce que** ladite deuxième électrode comprend deux demi-électrodes (16, 17), une desdites demi-électrodes étant mobile par rapport à l'autre de manière à pouvoir être écartée et rapprochée de cette dernière.

8. Installation selon la revendication 6 ou 7, **caractérisée en ce qu'**elle comprend un moyen pour refroidir ladite première électrode.

9. Installation selon l'une quelconque des revendications 6 à 8, **caractérisée en ce que** le canal intérieur (11) dudit conduit comportant une membrane (14) d'étanchéité sécable, la dimension longitudinale séparant la ou chaque gorge (15) de ladite première électrode de son extrémité libre est au moins égale à la distance devant séparer ladite membrane d'étanchéité du ou de chaque bourrelet (19) pour s'assurer de l'étanchéité de l'assemblage dudit conduit et du trocart correspondant.

## Patentansprüche

1. Verfahren zur Herstellung eines Anschlusses für einen Behälter zur medizinischen Verwendung, der zur Verabreichung einer Flüssigkeit bestimmt ist, wobei der Anschluss einen Hauptkörper, der einen inneren Kanal (11) für den Durchgang einer Flüssigkeit definiert, und mindestens einen durchgehenden oder unterbrochenen Wulst (19) enthält, der einen Vorsprung ins Innere des Kanals bildet, das mindestens die folgenden Schritte enthält:
a) es wird eine Rohrleitung (12) genommen, die keinen einen Vorsprung ins Innere des Kanals bildenden Wulst (19) aufweist, des inneren Kanals, in dem eine teilbare Dichtungsmembran gebildet wird, um ihn zu verschließen oder von ihr verschlossen zu werden,
b) in die so gebildete Rohrleitung (12) wird zumindest teilweise ein Dorn eingeführt, der an seiner Außenfläche mindestens eine durchgehende oder unterbrochene Ringnut (15) aufweist, deren Abmessungen und Form denjenigen eines durchgehenden oder unterbrochenen Wulsts (19) entsprechen, der im inneren Kanal (11) der Leitung geformt werden soll, wobei die Dichtungsmembran im inneren Kanal (11) positioniert wird, um einen Anschlag zu bilden, der es ermöglicht, die Verschiebung des Dorns bei seiner Einführung in den Kanal zu stoppen, um den oder jeden Wulst (19) an einem präzisen Punkt oder präzisen Punkten des Kanals zu positionieren,
c) die Rohrleitung (12) wird nur zum Teil erwärmt, wobei der Teil mindestens den Abschnitt der Leitung enthält, der sich im Bereich der mindestens einen Nut (15) befindet, wenn der Dorn in der Rohrleitung (12) positioniert wurde, so dass das Material der Rohrleitung (12) die mindestens eine Nut (15) füllt.

2. Verfahren nach Anspruch 1, **dadurch gekennzeichnet, dass** der Dorn vor seinem Herausziehen aus dem inneren Kanal (11) der Rohrleitung (12) abgekühlt wird.

3. Verfahren nach Anspruch 1 oder 2, **dadurch gekennzeichnet, dass** im Schritt c) durch ein Heizverfahren erwärmt wird, das aus der Gruppe ausgewählt wird, die eine dielektrische Erwärmung, eine Strahlungserwärmung und Erwärmung durch direkten Kontakt mit erwärmten Elementen enthält.

4. Verfahren nach Anspruch 3, **dadurch gekennzeichnet, dass** bei der Anwendung eines dielektrischen Erwärmungsverfahrens der Dorn als eine der Elektroden verwendet wird.

5. Verfahren nach einem der Ansprüche 1 bis 4, **dadurch gekennzeichnet, dass** im Schritt a) eine Abschrägung an mindestens einem der Enden der Körperleitung hergestellt wird, so dass mindestens eines der Enden abgeschrägt ist, und/oder an der Außenfläche der Körperleitung mindestens eines der Elemente gebildet wird, die aus der Gruppe ausgewählt werden, die enthält: eine oder mehrere vorstehend angeordnete Ringverdickungen, ein Verbindungelement, das in den biegsamen Behälter integriert sein kann, wobei dieses Verbindungselement an jedem seiner Seitenränder eine Rippe aufweist, wobei diese Rippen je eine Verlängerung des zentralen Teils dieses Verbindungselements bilden.

6. Anlage zur Durchführung des Verfahrens zur Herstellung eines Anschlusses nach einem der Ansprüche 1 bis 5, wobei die Anlage enthält
- einen elektrischen Hochfrequenzgenerator, der fähig ist, eine Hochfrequenzspannung zu liefern,
- eine erste rohrförmige Elektrode (10) mit einem Durchmesser gleich oder im Wesentlichen gleich dem Durchmesser des inneren Kanals (11) der Rohrleitung (12), um in den inneren Kanal (11) eingeführt werden zu können, wobei die erste Elektrode an ihrer Außenfläche mindestens eine durchgehende oder unterbrochene Ringnut (15) aufweist, deren Abmessungen und Form denjenigen eines durchgehenden oder unterbrochenen Wulsts (19) entsprechen, der im inneren Kanal (11) der Leitung gebildet werden soll,
- eine zweite Elektrode, die ein Loch eines Durchmessers kleiner als der oder gleich dem Außendurchmesser der Rohrleitung (12) aufweist, um eine Aufnahme zu definieren, um die Leitung aufzunehmen und zum Teil zu umgeben, und dass
- da der innere Kanal (11) der Leitung eine teilbare Dichtungsmembran (14) aufweist, das freie Ende (13) der ersten rohrförmigen Elektrode (10) eine nicht spitze Kontaktfläche aufweist, die es ermöglicht, diese gegen die Dichtungsmembran (14) anzupressen, ohne sie zu zerreißen.

7. Anlage nach Anspruch 6, **dadurch gekennzeichnet, dass** die zweite Elektrode zwei Halbelektroden (16, 17) enthält, wobei eine der Halbelektroden bezüglich der anderen beweglich ist, so dass sie von dieser letzteren entfernt und an sie angenähert werden kann.

8. Anlage nach Anspruch 6 oder 7, **dadurch gekennzeichnet, dass** sie eine Einrichtung zum Kühlen der ersten Elektrode enthält.

9. Anlage nach einem der Ansprüche 6 bis 8, **dadurch gekennzeichnet, dass**, da der innere Kanal (11) der Leitung eine teilbare Dichtungsmembran (14) enthält, die die oder jede Nut (15) der ersten Elektrode von ihrem freien Ende trennende Längsabmessung mindestens gleich dem Abstand ist, der die Dichtungsmembran von dem oder jedem Wulst (19) trennen muss, um die Dichtheit des Zusammenbaus der Leitung und des entsprechenden Trokars zu gewährleisten.

## Claims

1. Method for manufacturing a connector for a container for medical use intended for the administration of fluid, said connector comprising a main body defining an inside channel (11) for a fluid to pass through and at least one continuous or discontinuous flange (19), projecting inside said channel, comprising at least the following steps:
a) a tubular duct (12) with no flange (19) projecting inside said channel is taken as a starting point, said channel inside which is formed to block it, or being blocked by, a divisible sealing membrane,
b) inserted at least partially into said tubular duct (12) formed in this manner is a mandrel having, on the outer surface thereof, at least one continuous or discontinuous annular recess (15), the dimensions and the shape of which correspond to those of a continuous or discontinuous flange (19) to be formed in the inside channel (11) of said duct, said sealing membrane being positioned in said inside channel (11) in order to form an abutment for stopping the movement of the mandrel when it is inserted into said channel in order to position said or each flange (19) at one or more precise points of said channel,
c) only said tubular duct (12) is partially heated, said part comprising at least said duct portion placed at said at least one recess (15) when said mandrel has been positioned in said tubular duct (12), such that the material of said tubular duct (12) fills said at least one recess (15).

2. Method according to Claim 1, **characterized in that** said mandrel is cooled before removing the latter from the inside channel (11) of said tubular duct (12).

3. Method according to Claim 1 or 2, **characterized in that**, in step c), heating takes place using a heating method chosen from dielectric heating, radiation and direct contact with heated elements.

4. Method according to Claim 3, **characterized in that**, when implementing a dielectric heating method, said mandrel is used as one of said electrodes.

5. Method according to any one of Claims 1-4, **characterized in that**, in step a), a chamfer is produced at at least one of the ends of said body duct such that at least one of said ends is bevelled and/or on the outer surface of said body duct is formed at least one of the elements chosen from the group comprising one or more projecting rings, a joining element which can be incorporated in the flexible container, this joining element including, at each of the side edges thereof, a blade, these blades each forming an extension of the central part of this joining element.

6. Apparatus for implementing the method for manufacturing a connector according to any one of Claims 1-5, the apparatus comprising:
- a high-frequency electric generator suitable for providing a high-frequency voltage,
- a first tubular electrode (10) having a diameter equal or substantially equal to the diameter of the inside channel (11) of said tubular duct (12) such as to be able to be inserted into said inside channel (11), said first electrode including, on the outer surface thereof, at least one continuous or discontinuous annular recess (15), the dimensions and the shape of which correspond to those of a continuous or discontinuous flange (19) to be formed in the inside channel (11) of said duct,
- a second electrode including a hole having a diameter less than or equal to the outer diameter of said tubular duct (12) such as to define a housing for receiving and surrounding, in part, said duct, and in that
- since the inside channel (11) of said duct includes a divisible sealing membrane (14), the free end (13) of said first tubular electrode (10) has a blunt contact surface for placing it flush against said sealing membrane (14) without tearing it.

7. Apparatus according to Claim 6, **characterized in that** said second electrode comprises two half-electrodes (16, 17), one electrode of said half-electrodes being movable in relation to the other such as to be able to be moved apart from and brought closer to the latter.

8. Apparatus according to Claim 6 or 7, **characterized in that** it comprises a means for cooling said first electrode.

9. Apparatus according to any one of Claims 6-8, **characterized in that**, since the inside channel (11) of said duct includes a divisible sealing membrane (14), the longitudinal dimension separating the or each recess (15) of said first electrode and the free end thereof is at least equal to the distance that is to separate said sealing membrane and the or each flange (19) in order to seal the assembly of said duct and of the corresponding puncture needle.
